# EUROPÄISCHE PATENTSCHRIFT

(11) **EP 0 815 097 B1**
(45) Veröffentlichungstag und Bekanntmachung des Hinweises auf die Patenterteilung: **20.10.1999**
(21) Anmeldenummer: 96910004.9
(22) Anmeldetag: 21.03.1996
(51) Int. Cl.: C07D 307/08, C07C 29/141

(54) **Verfahren zur Herstellung von 1,4-Butandiol und Tetrahydrofuran aus Furan**
Method of producing 1,4-butanediol and tetrahydrofuran from furan
Procédé de fabrication de 1,4-butanediol et de tétrahydrofuranne à partir de furanne

(30) Priorität: 22.03.1995 DE 19510438
(43) Veröffentlichungstag der Anmeldung: 07.01.1998
(73) Patentinhaber: BASF AKTIENGESELLSCHAFT, 67056 Ludwigshafen (DE)
(72) Erfinder: FISCHER, Rolf, D-69121 Heidelberg (DE); PINKOS, Rolf, D-67098 Bad Dürkheim (DE)
(74) Vertreter: Isenbruck, Günter, Dr.
(86) Internationale Anmeldenummer: EP9601247
(87) Internationale Veröffentlichungsnummer: WO9629322

(56) Entgegenhaltungen:
- US-A- 3 021 342
- US-A- 4 146 741
- US-A- 4 475 004
- US-A- 4 476 332
- CHEMICAL ABSTRACTS, vol. 53, no. 14, 25.Juli 1959 Columbus, Ohio, US; abstract no. 13060h, Spalte 13060; XP002008811 & SU,A,114 928 (P.A. MOSHKIN, ET AL.) in der Anmeldung erwähnt
- INDUSTRIAL AND ENGINEERING CHEMISTRY, PRODUCT RESEARCH AND DEVELOPMENT, Bd. 12, Nr. 4, 1973, WASHINGTON, DC, US, Seiten 310-311, XP002008812 J.M WATSON: "Butane-1,4-diol from hydrolytic reduction of furan" in der Anmeldung erwähnt

## Beschreibung

Die vorliegende Erfindung betrifft ein Verfahren zur Herstellung von 1,4-Butandiol/Tetrahydrofuran (THF) aus Furan.

In dem russischen Patent 114 928 wird Furan in neutraler oder schwach saurer, wäßriger Lösung (Ameisensäurezusatz) an Nickelkatalysatoren zu einem Gemisch von Tetrahydrofuran, 1,4-Butandiol und kleinen Mengen n-Butanol hydriert. Die Ausbeuten an Butandiol/THF sind bei geringen Umsätzen bescheiden. J.M. Watson, Ind. Eng. Chem. Prod. Res. Develop 12 (4), 310 - 311 (1973) beschreibt die gleiche Umsetzung an Nickelkatalysatoren, jedoch in Gegenwart von Essigsäure. In US 4 146 741 wird ebenfalls die Hydrierung von Furan zu Butandiol an Nickelkatalysatoren, die auch Kupfer enthalten können, beschrieben, aber in Anwesenheit von wäßrigen Dicarbonsäurelösungen. Außerdem wird ausgesagt, daß bei zu geringer Acidität des Reaktionssystems fast nur THF, aber nur noch wenig Diol gebildet wird. In US 4 475 004 finden neben Nickelkatalysatoren halogenierte Carbonsäuren Verwendung. In Abwesenheit dieser halogenierten Carbonsäuren wird Butandiol nur in Spuren gebildet. Die gleichen halogenierten Carbonsäuren werden auch in US 4 476 332 in Kombination mit Rutheniumkatalysatoren angewendet. Ohne diese Säurepromotoren wird kein Butandiol gebildet.

Die vorgenannten Methoden zur Herstellung von 1,4-Butandiol und THF aus Furan haben den Nachteil, daß sie meist nur geringe Ausbeuten an Butandiol ergeben und Nebenprodukte entstehen (z.B. Butandioldiacetat), die schlecht vom Wertprodukt abtrennbar sind. Generell gilt, daß die eine Katalysatorkomponente (Carbonsäure) die kontinuierliche Ausführung der oben beschriebenen Umsetzungen im technischen Maße erheblich erschwert. Denn einerseits müssen Vorkehrungen getroffen werden, um die Korrosion der Anlagen zu vermeiden (die Verwendung von Halogencarbonsäuren ist praktisch prohibitiv). Andererseits müssen die Säuren mit dem Produktstrom ausgetragen und nach aufwendiger Abtrennung ständig rückgeführt werden. Da beim Vorliegen einer Säure und eines Alkohols (Butandiol) zu einem gewissen Maß immer Veresterung eintritt, muß ein Teil der Carbonsäure ständig ergänzt werden. Außerdem ist während der Reaktion mit der Hydrierung der Säure zu rechnen, was ebenfalls zu ständigen Verlusten an Carbonsäuren führt.

Es lag deshalb die Aufgabe vor, ein Verfahren zu entwickeln, das einerseits zu hohen Selektivitäten an Butandiol/THF führt, andererseits ohne Promotoren wie die erwähnten Carbonsäuren auskommt.

Dementsprechend wurde ein Verfahren zur Herstellung von 1,4-Butandiol/THF aus Furan entwickelt, das dadurch gekennzeichnet ist, daß man Furan einstufig als Reaktionsmischung mit Wasser und in Gegenwart von Wasserstoff, jedoch in Abwesenheit einer in Wasser löslichen Säure oder Carbonsäure, bei einer Temperatur von 20 - 300 °C und einem Druck von 100 kPa - 30 MPa (1 - 300 bar) an einem Hydrierkatalysator umsetzt. Dieser Hydrierkatalysator enthält in Form einer Verbindung, vorzugsweise als Oxid, oder elementar mindestens eines der Elemente der I., V., VI., VII. oder VIII. Nebengruppe des Periodensystems der Elemente, wobei die zusätzliche Einschränkung gilt, daß er nicht Nickel allein enthält.

Bei Durchführung des erfindungsgemäßen Verfahrens wird Furan mit Wasser im allgemeinen in einem Molverhältnis Furan/Wasser von 1 : 0,1 bis 1 : 100, vorzugsweise von 1 : 0,2 bis 1 : 50 und besonders bevorzugterweise von 1 : 1 bis 1 : 10, zu 1,4-Butandiol/THF umgesetzt. Dies erfolgt in Gegenwart von Wasserstoff und einem Hydrierkatalysator bei einem Druck von im allgemeinen 100 kPa - 30 MPa (1 - 300 bar), vorzugsweise von 500 kPa - 20 MPa (5 - 200 bar) und besonders bevorzugterweise von 1 - 15 MPa (10 - 150 bar), und einer Temperatur von 20 bis 300°C, vorzugsweise 40 bis 230°C und besonders bevorzugterweise 80 bis 200°C.

Es werden nach dem erfindungsgemäßen Verfahren, bezogen auf die Masse, Butandiol : THF-Ausbeuteverhältnisse von im allgemeinen 0,1 - 40, bevorzugterweise 0,2 - 35, besonders bevorzugterweise 0,5 - 25, erhalten.

Als Hydrierkatalysatoren können für das erfindungsgemäße Verfahren im allgemeinen alle zur Hydrierung von Carbonylgruppen geeigneten Katalysatoren, die mindestens eines der Elemente der I., V., VI., VII. oder VIII. Nebengruppe des Periodensystems (außer Ni alleine) enthalten, Verwendung finden. Die Katalysatoren können dabei fest angeordnet, suspendiert oder homogen gelöst sein.

Das katalytisch aktive Metall der I. und/oder V. und/oder VI. und/oder VII, und/oder VIII. (außer Ni alleine) Nebengruppe bzw. seine Verbindung kann mit weiteren Metallen/Elementen der I. und/oder V. und/oder VI. und/oder VII. und/oder VIII. Nebengruppe und/oder III. und/oder IV. Hauptgruppe gemischt oder legiert vorliegen.

Bevorzugt werden Heterogenkatalysatoren, die vorzugsweise als Metalle in aktivierter, feinverteilter Form mit großer Oberfläche vorliegen. Bevorzugte Beispiele dafür sind ein Ru-Schwamm oder Mischungen zweier oder mehrerer Metalle, wie sie z.B. durch gemeinsame Reduktion von entsprechenden Metallsalzen und/oder anderen Metallverbindungen entstehen, insbesondere ein Re/Ru-Schwamm nach Reduktion einer wäßrigen Re₂O₇/RuCl₃-Lösung,

Des weiteren können für das erfindungsgemäße Verfahren vorteilhafterweise sogenannte Fällungskatalysatoren verwendet werden. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder -Carbonat-Lösungen, als z.B, schwerlösliche Hydroxide, Oxidhydrate, basische Salze oder Carbonate ausfällt. Die erhaltenen Niederschläge werden anschließend getrocknet und dann durch Calcinierung bei im allgemeinen 300 bis 700°C in die entsprechenden Oxide, Mischoxide und/oder gemischtvalentigen Oxide umgewandelt, welche durch eine Behandlung mit Wasserstoff oder mit Wasserstoff enthaltenden Gasen bei in der Regel 100 bis 400 °C zu den betreffenden Metallen und/oder oxidischen Verbindungen niederer Oxidationsstufe reduziert und in die eigentliche, katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhafterweise aber auch gleichzeitig mit dem Trägermaterial aus den betreffenden Salzlösungen gefällt werden.

Es ist bevorzugt, für das erfindungsgemäße Verfahren Hydrierkatalysatoren zu verwenden, welche die die Hydrierung katalysierenden Metalle oder Metallverbindungen als Abscheidung auf einem Trägermaterial enthalten. Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen solche Trägerkatalysatoren, bei denen die katalytisch-hydrierend wirkenden Komponenten z.B. durch Imprägnierung auf ein Trägermaterial aufgebracht worden sind.

Die Art der Aufbringung der katalytisch aktiven Metalle bzw. Metallverbindungen auf den Träger ist in der Regel nicht kritisch und kann auf verschiedenerlei Art und Weise geschehen. Die katalytisch aktiven Metalle bzw. Metallverbindungen können z.B. durch Tränkung des Trägermaterials mit Lösungen oder Suspensionen der Salze oder Oxide der betreffenden Elemente, Trocknung und anschließende Reduktion der Metallverbindung zu den betreffenden Metallen oder Verbindungen niederer Oxidationsstufen mittels eines Reduktionsmittels, vorzugsweise Wasserstoff oder komplexer Hydride, auf diese Trägermaterialien aufgebracht werden. Eine andere Möglichkeit zur Aufbringung der katalytisch aktiven Metalle bzw. Metallverbindungen auf den Träger besteht darin, diesen mit einer Lösung thermisch leicht zersetzbarer Komplexverbindungen, z.B. mit Carbonyl- oder Hydrido-Komplexen der katalytisch aktiven Metalle, zu imprägnieren und den so getränkten (imprägnierten) Träger zwecks thermischer Zersetzung der absorbierten Metallverbindungen auf Temperaturen von z.B. 300 bis 600°C zu erhitzen. Weiterhin können die katalytisch aktiven Metalle bzw. Metallverbindungen durch Aufdampfung oder durch Flammspritzen auf dem Katalysatorträger abgeschieden werden.

Der Gehalt dieser Trägerkatalysatoren an den katalytisch aktiven Metallen/Metallverbindungen ist prinzipiell für das Gelingen des erfindungsgemäßen Verfahrens nicht kritisch. Es versteht sich für den Fachmann von selbst, daß höhere Gehalte dieser Trägerkatalysatoren an katalytisch aktiven Metallen/Metallverbindungen zu höheren Raum-Zeit-Ausbeuten führen als niedrigere Gehalte. Im allgemeinen werden aber Trägerkatalysatoren verwendet, deren Gehalt an katalytisch aktiven Metallen/Metallverbindungen, bezogen auf den gesamten Katalysator, 0,1 bis 80 Gew.-%, vorzugsweise 0.5 bis 30 Gew.-%, beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch über- oder unterschritten werden, ohne daß sich dies nachteilig auf das Ergebnis des erfindungsgemäßen Verfahrens auswirkt. Bevorzugterweise sind mehrere der katalytisch aktiven Metalle/Metallverbindungen auf dem jeweiligen Trägermaterial aufgebracht. Diese können dabei gleichzeitig oder auch nacheinander auf den Träger aufgebracht werden. Weiterhin können die katalytisch aktiven Metalle/Metallverbindungen beispielsweise nach den Verfahren auf den Träger aufgebracht werden, die aus den DE-A 25 19 817, EP-A 0 147 219 und EP-A 0 285 420 bekannt sind. Bei den Katalysatoren vorgenannter Schriften liegen die katalytisch aktiven Metalle/Metallverbindungen als Legierungen vor, die durch thermische Behandlung und/oder Reduktion der z.B. durch Tränkung aufgebrachten, zuvor erwähnten Salze oder Komplexe erzeugt werden.

Weitere Varianten bevorzugter Katalysatorsysteme sind z.B. in JP 59 184 138 und DE 42 00 247.8 beschrieben. Dabei werden in Wasser lösliche oder nichtlösliche, anorganische Hilfsstoffe mit in das System eingebracht (z.B. Zinkoxid, Zinksulfat, Zinkhydroxid oder Nickelsulfat).

Die Aktivierung sowohl der Fällungskatalysatoren als auch der Trägerkatalysatoren kann zu Beginn der Reaktion durch den anwesenden Wasserstoff in situ erfolgen. Bevorzugterweise werden diese Katalysatoren jedoch vor ihrer Verwendung separat aktiviert.

Als Trägermaterialien können im allgemeinen die Oxide des Aluminiums und Titans, Zirkoniumdioxid, Siliciumdioxid, Tonerden wie Montmorillonite, Silikate wie Magnesium- oder Aluminiumsilikate, Zeolithe wie ZSM-5- oder ZSM-10-Zeolithe sowie Aktivkohle verwendet werden. Bevorzugte Trägermaterialien sind Aluminiumoxide, Titanoxide, Siliciumdioxid, Zirkoniumdioxid und Aktivkohle. Selbstverständlich können auch Mischungen verschiedener Trägermaterialien als Träger für die im erfindungsgemäßen Verfahren anwendbaren Katalysatoren dienen. Besonders bevorzugterweise werden Aktivkohle-Träger eingesetzt.

Als für das erfindungsgemäße Verfahren verwendbare Heterogenkatalysatoren seien die folgenden beispielhaft genannt:

Ruthenium-Schwamm, Rhenium-Schwamm, Ruthenium/Rhenium-Schwamm, Ruthenium auf Aktivkohle, Rhenium auf Aktivkohle. Rhenium auf Siliciumdioxid, Rhenium/Zinn auf Aktivkohle, Ruthenium/Palladium auf Aktivkohle, Rhenium/Kupfer auf Aktivkohle, Rhenium/Nickel auf Aktivkohle, Ruthenium/Nickel auf Aktivkohle, Rhenium/Ruthenium auf Aktivkohle, Kobalt/Rhenium auf Aktivkohle, Ruthenium/Kupfer auf Aktivkohle, Kupfer/Nickel auf Aktivkohle, Kupfer/Ruthenium auf Aktivkohle, Kupfer auf Aktivkohle, Kupfer auf Siliciumdioxid, Kupfer auf Aluminiumoxid, Kupferchromit sowie Bariumkupferchromit.

Als Homogenkatalysatoren können solche verwendet werden, wie sie beispielsweise in Houben-Weyl, Methoden der organischen Chemie, Band IV/lc, S. 45 bis 67, Thieme Verlag, Stuttgart 1980, beschrieben werden. Bevorzugte Homogenkatalysatoren sind insbesondere die Komplexe des Rhodiums, Rutheniums und Kobalts mit Phosphin- oder Phosphit-Liganden, deren Herstellung z.B. in CA 72 76 41, H. Brunner in Hartley: The Chemistry of the Metal-Carbon Bond: Bd. 5, S. 110 - 124, John Wiley u. Sons. New York, sowie in Toth et al.. Inorg. Chim. Acta 42, 153 (1980), und der dort zitierten Literatur beschrieben ist.

Das erfindungsgemäße Verfahren kann sowohl kontinuierlich als auch diskontinuierlich ausgeübt werden. Zur kontinuierlichen Betriebsweise können vorteilhafterweise beispielsweise Rohrreaktoren eingesetzt werden, in denen der Katalysator vorteilhafterweise in Form eines Festbetts angeordnet ist, über das die Reaktionsmischung in Sumpf- oder Rieselfahrweise geleitet werden kann.

Bei der diskontinuierlichen Betriebsweise können einfache Rührreaktoren verwendet werden. Bevorzugt ist jedoch die Verwendung von Schlaufenreaktoren. Dabei wird der Katalysator zweckmäßigerweise in Form eines Festbetts angeordnet. Bei nicht-vollständiger Umsetzung des Ausgangsmaterials kann dieses zweckmäßigerweise entweder nach destillativer Abtrennung der Wertprodukte oder als Teilstrom zusammen mit den anderen Reaktionsprodukten in die Umsetzung zurückgeführt werden. Dies erweist sich insbesondere bei der kontinuierlichen Betriebsweise als vorteilhaft.

Das erfindungsgemäße Verfahren kann vorteilhafterweise in Anwesenheit eines (unter den Reaktionsbedingungen) inerten Lösungsmittels durchgeführt werden, beispielsweise mit wasserlöslichen Ethern wie Tetrahydrofuran, Dioxan oder Diethylenglycoldimethylether. Vorteilhafterweise können auch Alkohole, insbesondere das Verfahrensprodukt 1,4-Butandiol, als Lösungsmittel eingesetzt werden.

Der Reaktionsaustrag des erfindungsgemäßen Verfahrens wird zweckmäßigerweise destillativ aufgearbeitet, wobei außer den Verfahrensprodukten 1,4-Butandiol und THF die als Nebenprodukte anfallenden Verbindungen gamma-Butyrolacton und n-Butanol als weitere Wertprodukte gewonnen werden können.

Das Ausgangsmaterial Furan kann beispielsweise durch Decarbonylierung von Furfural leicht hergestellt werden.

1,4-Butandiol wird weltweit in großem Umfang hergestellt und dient als Diolkomponente zur Herstellung von u.a. Polyestern, Polyurethanen und Epoxyharzen. THF wird z.B. als Lösungsmittel oder für die Herstellung von Poly-THF verwendet.

Weitere bevorzugte Ausführungsformen der vorliegenden Erfindung sind nachfolgend in den Beispielen sowie den abhängigen Ansprüchen angegeben.

### Beispiele

### Beispiel 1

In einen 50 ml Metallautoklaven mit Rührer wurden 0,5 g eines Rhenium/Ruthenium/Aktivkohle-Katalysators (Rhenium: 5 Gew.-%, Ruthenium: 1 Gew.-%, jeweils bezogen auf das Gesamtgewicht des Katalysators), 1.3 g Furan und 5 g Wasser gefüllt. Der Katalysator war zuvor durch Tränken der Aktivkohle mit einer wäßrigen Re₂O₇/RuCl₃-Lösung, Trocknung bei 120°C und zweistündige Aktivierung im Wasserstoffstrom bei 300°C hergestellt worden. Durch Einpressen von Wasserstoff wurde im Autoklaven ein Druck von 3 MPa (30 bar) erzeugt. Dann wurde der Autoklav für drei Stunden auf eine Temperatur von 160°C aufgeheizt. Danach wurde der Autoklav auf Raumtemperatur abgekühlt und bei einem Restdruck von ca. 1,8 MPa (18 bar) entspannt. Bei einem Umsatz von 99% wurden die folgenden gaschromatographisch ermittelten Ausbeuten erhalten: 24% THF, 68% 1,4-Butandiol, 2% Butyrolacton und 5% n-Butanol (diese %-Angaben sind, ebenso wie die in allen nachfolgenden Beispielen, Gew.-%-Angaben).

### Beispiel 2

Analog Beispiel 1 wurden 1,35 g Furan an einem Ru/Aktivkohle-Katalysator (Ruthenium: 1 Gew.-%, bezogen auf das Gesamtgewicht des Katalysators, hergestellt durch Tränken der Aktivkohle mit einer wäßrigen RuCl₃-Lösung, Trocknung und Aktivierung wie in Beispiel 1) bei einer Verweilzeit von 1,5 h umgesetzt. Bei einem Umsatz von 97% wurden die folgenden gaschromatographisch ermittelten Ausbeuten erhalten: 51% THF, 33% 1,4-Butandiol, 12% n-Butanol.

### Beispiel 3

Analog Beispiel 1 wurden 1,52 g Furan an einem Co/Re/Aktivkohle-Katalysator (Kobalt: 3 Gew.-%, Rhenium: 3 Gew.-%, jeweils bezogen auf das Gesamtgewicht des Katalysators, hergestellt durch Tränken der Aktivkohle mit einer wäßrigen Co(OAc)₂/Re₂O₇-Lösung. Trocknung und Aktivierung wie in Beispiel 1) bei einer Verweilzeit von 2 h umgesetzt. Bei einem Umsatz von 80% wurden die folgenden gaschromatographisch ermittelten Ausbeuten erhalten: 30% THF, 33% 1,4-Butandiol. 10% n-Butanol und 1% Butyrolacton.

### Beispiel 4

Analog Beispiel 1 wurden 1,4 g Furan an einem Ru/Cu/Aktivkohle-Katalysator (Ruthenium: 3 Gew.-%, Kupfer: 3 Gew.-%, jeweils bezogen auf das Gesamtgewicht des Katalysators, hergestellt durch Tränken der Aktivkohle mit einer wäßrigen RuCl₃/Cu(OAc)₂-Lösung, Trocknung und Aktivierung wie in Beispiel 1) bei einer Verweilzeit von 3 h umgesetzt. Bei einem Umsatz von 89% wurden die folgenden gaschromatographisch ermittelten Ausbeuten erhalten: 50% THF, 31% 1,4-Butandiol, 5% n-Butanol und 1% Butyrolacton.

### Beispiel 5

Analog Beispiel 1 wurden 1,4 g Furan an einem Re/Ni/Aktivkohle-Katalysator (Rhenium: 5 Gew.-%, Nickel: 5 Gew.-%, jeweils bezogen auf das Gesamtgewicht des Katalysators, hergestellt durch Tränken der Aktivkohle mit einer wäßrigen Re₂O₇/Ni(OAc)₂-Lösung, Trocknung und Aktivierung wie in Beispiel 1) bei einer Verweilzeit von 2 h umgesetzt. Bei einem Umsatz von 99% wurden die folgenden gaschromatographisch ermittelten Ausbeuten erhalten: 58% THF, 36% 1,4-Butandiol, 3% n-Butanol und 1% Butyrolacton.

### Beispiel 6

Analog Beispiel 1 wurden 1.44 g Furan an einem Rh/Re/Aktivkohle-Katalysator (Rhodium: 1 Gew.-%, Rhenium: 5 Gew.-%, jeweils bezogen auf das Gesamtgewicht des Katalysators, hergestellt durch Tränken der Aktivkohle mit einer wäßrigen RhCl₃/Re₂O₇-Lösung, Trocknung und Aktivierung wie in Beispiel 1) bei einer Verweilzeit von 0,75 h umgesetzt. Bei einem Umsatz von 100% wurden die folgenden gaschromatographisch ermittelten Ausbeuten erhalten: 75% THF, 18% 1,4-Butandiol, 2% n-Butanol und 1% Butyrolacton.

### Beispiel 7

Analog Beispiel 1 wurden 1,52 g Furan an einem Ni/Cu/Aktivkohle-Katalysator (Nickel: 3 Gew.-%, Kupfer: 3 Gew.-%, jeweils bezogen auf das Gesamtgewicht des Katalysators, hergestellt durch Tränken der Aktivkohle mit einer wäßrigen Ni(OAc)₂/Cu(OAc)₂-Lösung, Trocknung und Aktivierung wie in Beispiel 1) bei einer Verweilzeit von 1,5 h umgesetzt. Bei einem Umsatz von 100% wurden die folgenden gaschromatographisch ermittelten Ausbeuten erhalten: 65% THF, 32% 1,4-Butandiol, 1% n-Butanol und 1% Butyrolacton.

### Beispiel 8

Analog Beispiel 1 wurden 1,4 g Furan an einem Ru/Ni/Aktivkohle-Katalysator (Ruthenium: 1 Gew.-%, Nickel: 4 Gew.-%, jeweils bezogen auf das Gesamtgewicht des Katalysators, hergestellt durch Tränken der Aktivkohle mit einer wäßrigen RuCl₃/Ni(OAc)₂-Lösung. Trocknung und Aktivierung wie in Beispiel 1) bei einer Verweilzeit von h umgesetzt. Bei einem Umsatz von ca. 60% wurden die folgenden gaschromatographisch ermittelten Ausbeuten erhalten: 48% THF, 10% 1,4-Butandiol und 2% n-Butanol.

### Beispiel 9

Der Versuch aus Beispiel 8 wurde in Gegenwart von 1 g NiSO₄ x 6 H₂O wiederholt. Bei einem Umsatz von 100% wurden die folgenden gaschromatographisch ermittelten Ausbeuten erhalten: 20% THF, 70% 1,4-Butandiol und 7% n-Butanol.

### Beispiel 10

Analog Beispiel 1 wurden 1,5 g Furan an einem Ru/Cu/Re/Aktivkohle-Katalysator (Ruthenium: 1 Gew.-%, Kupfer: 3 Gew.-%, Rhenium: 5 Gew.-%, jeweils bezogen auf das Gesamtgewicht des Katalysators, hergestellt durch Tränken der Aktivkohle mit einer wäßrigen RuCl₃/Cu(OAc)₂/Re₂O₇-Lösung, Trocknung und Aktivierung wie in Beispiel 1) bei 170°C und einer Verweilzeit von 3 h umgesetzt. Bei einem Umsatz von 98% wurden die folgenden gaschromatographisch ermittelten Ausbeuten erhalten: 35% THF, 53% 1,4-Butandiol, 7% n-Butanol und 3% Butyrolacton.

## Patentansprüche

1. Verfahren zur Herstellung von 1,4-Butandiol und THF aus Furan, dadurch gekennzeichnet, daß Furan als Reaktionsmischung mit Wasser und in Gegenwart von Wasserstoff, aber in Abwesenheit einer in Wasser löslichen Säure, einstufig an einem Hydrierkatalysator umgesetzt wird, wobei der Hydrierkatalysator mindestens ein Element der I., V., VI., VII. oder VIII. Nebengruppe in Form einer Verbindung oder elementar enthält, und wobei die Einschränkung gilt, daß der Katalysator nicht Nickel allein enthält.

2. Verfahren nach Anspruch 1, dadurch gekennzeichnet, daß die Umsetzung bei 20 - 300°C, bevorzugterweise bei 40 - 230°C, besonders bevorzugterweise bei 80 - 200°C, erfolgt.

3. Verfahren nach Anspruch 1 oder 2, dadurch gekennzeichnet, daß die Umsetzung bei einem Druck von 0,1 - 30 MPa, bevorzugterweise bei einem Druck von 0,5 - 20 MPa, besonders bevorzugterweise bei einem Druck von 1 - 15 MPa, erfolgt.

4. Verfahren nach einem der vorhergehenden Ansprüche, dadurch gekennzeichnet, daß die Edukte Furan und Wasser in einem Molverhältnis von 1 : 0,1 - 100, bevorzugterweise in einem Molverhältnis von 1 : 0,2 - 50, besonders bevorzugterweise in einem Molverhältnis von 1 : 1 - 10, eingesetzt werden.

5. Verfahren nach einem der vorhergehenden Ansprüche, dadurch gekennzeichnet, daß der mindestens ein Nebengruppenelement enthaltende Katalysator außerdem ein Element der III. oder IV. Hauptgruppe enthält.

6. Verfahren nach einem der vorhergehenden Ansprüche, dadurch gekennzeichnet, daß der Katalysator durch Imprägnierung, Fällung oder Tränkung auf ein Trägermaterial aufgebracht worden ist, wobei dieses Trägermaterial ein Oxid des Aluminiums oder Titans. ZrO₂, SiO₂, Tonerden wie Montmorillonite, Silikate wie Mg- oder Al-Silikat, Zeolithe wie ZSM-5- oder ZSM-10-Zeolith sowie Aktivkohle sein kann.

7. Verfahren nach einem der vorhergehenden Ansprüche, dadurch gekennzeichnet, daß der Katalysator Ruthenium enthält.

8. Verfahren nach einem der Ansprüche 1 - 6, dadurch gekennzeichnet, daß als Katalysator Mischungen oder Legierungen von Elementen der Nebengruppen VI und/oder VIII bzw. deren Verbindungen verwendet werden.

9. Verfahren nach einem der vorhergehenden Ansprüche, dadurch gekennzeichnet, daß der Katalysator vor seiner Verwendung aktiviert wird.

10. Verfahren nach einem der vorhergehenden Ansprüche, dadurch gekennzeichnet, daß die aktive Komponente des Katalysators, bezogen auf das Gesamtgewicht des Katalysators, 0.1 - 80 Gew.-%, vorzugsweise 0.5 - 30 Gew.-%, ausmacht.

## Claims

1. A process for the preparation of 1,4-butanediol and THF from furan, which comprises converting furan as a reaction mixture with water and in the presence of hydrogen but in the absence of a water-soluble acid in a single stage over a hydrogenation catalyst, the hydrogenation catalyst containing at least one element of subgroup I, V, VI, VII or VIII in the form of a compound or in elemental form and the restriction that the catalyst does not contain nickel alone being applicable.

2. A process as claimed in claim 1, wherein the reaction is carried out at 20 - 300°C, preferably 40 - 230°C, particularly preferably 80 - 200°C.

3. A process as claimed in claim 1 or 2, wherein the reaction is carried out at 0.1 - 30, preferably 0.5 - 20, particularly preferably 1 - 15 MPa.

4. A process as claimed in any of the preceding claims, wherein the starting materials furan and water are used in a molar ratio of 1:0.1 - 1:100, preferably 1:0.2 - 1:50, particularly preferably 1:1 - 1:10.

5. A process as claimed in any of the preceding claims, wherein the catalyst containing at least one subgroup element furthermore contains an element of main group III or IV.

6. A process as claimed in any of the preceding claims, wherein the catalyst has been applied to a carrier by impregnation or precipitation, and this carrier may be an oxide of aluminum or of titanium, ZrO₂, SiO₂, an alumina, such as a montmorillonite, a silicate, such as magnesium or aluminum silicate, a zeolite, such as ZSM-5 or ZSM-10 zeolite, or active carbon.

7. A process as claimed in any of the preceding claims, wherein the catalyst contains ruthenium.

8. A process as claimed in any of claims 1 to 6, wherein mixtures or alloys of elements of subgroups VI or VIII or compounds thereof are used as the catalyst.

9. A process as claimed in any of the preceding claims, wherein the catalyst is activated before being used.

10. A process as claimed in any of the preceding claims, wherein the active component of the catalyst accounts for 0.1 - 80, preferably 0.5 - 30, % by weight, based on the total weight of the catalyst.

## Revendications

1. Procédé de préparation de 1,4-butanediol et de THF à partir de furane caractérisé en ce que le furane est mis à réagir en tant que mélange réactionnel avec de l'eau et en présence d'hydrogène, mais en l'absence d'un acide soluble dans l'eau, en une étape sur un catalyseur d'hydrogénation, où le catalyseur d'hydrogénation contient au moins un élément des groupes secondaires IB, VB, VIB, VIIB ou VIII sous forme d'un composé ou sous forme élémentaire, et où le catalyseur ne contient pas du nickel seul.

2. Procédé selon la revendication 1, caractérisé en ce que la réaction est menée à 20-300°C, de préférence 40-230°C, de façon particulièrement préférée à 80-200°C.

3. Procédé selon la revendication 1 ou 2, caractérisé en ce que la réaction est menée sous une pression de 0,1-30 MPa, de préférence sous une pression de 0,5-20 MPa, de façon plus particulièrement préférée de 1-15 MPa.

4. Procédé selon l'une quelconque des revendications précédentes, caractérisé en ce que les produits de départ, le furane et l'eau, sont utilisés en un rapport molaire de 1:0,1-100, de préférence dans un rapport molaire de 1:0,2-50, de façon plus particulièrement préférée dans un rapport molaire de 1:1-10.

5. Procédé selon l'une quelconque des revendications précédentes, caractérisé en ce que le catalyseur contenant au moins un élément des groupes secondaires contient en outre un élément des groupes principaux IIIA ou IVA.

6. Procédé selon l'une quelconque des revendications précédentes, caractérisé en ce que le catalyseur est déposé sur un matériau support par imprégnation, précipitation ou immersion, où ce matériau support peut être un oxyde d'aluminium ou de titane, ZrO₂, SiO₂, des argiles comme la montmorillonite, des silicates comme le silicate de Mg ou de Al, des zéolithes comme ZSM-5 ou ZSM-10 ainsi que du charbon actif.

7. Procédé selon l'une quelconque des revendications précédentes, caractérisé en ce que le catalyseur contient du ruthénium.

8. Procédé selon l'une quelconque des revendications 1 à 6, caractérisé en ce que l'on utilise, en tant que catalyseur, des mélanges ou des alliages des éléments des groupes secondaires VIB et/ou VIII, ou de leurs composés.

9. Procédé selon l'une quelconque des revendications précédentes, caractérisé en ce que le catalyseur est activé avant son utilisation.

10. Procédé selon l'une quelconque des revendications précédentes, caractérisé en ce que les composants actifs du catalyseur représente 0,1-80% en poids, de préférence 0,5-30% en poids, par rapport au poids total du catalyseur.
